# EUROPEAN PATENT SPECIFICATION

(11) **EP 0 679 399 B1**
(45) Date of publication and mention of the grant of the patent: **06.08.1997**
(21) Application number: 95302497.3
(22) Date of filing: 13.04.1995
(51) Int. Cl.: A61K 31/665

(54) **Phosphate diesters for treatment of epidermis proliferative diseases**
Phosphatdiester zur Behandlung von proliferativen Erkrankungen der Epidermis
L'utilisation des diesters phosphatiques pour le traitement des maladies prolifératives de l'épiderme

(30) Priority: 22.04.1994 JP 84248/94
(43) Date of publication of application: 02.11.1995
(73) Proprietor: Senju Pharmaceutical Co., Ltd., Osaka-shi, Osaka 541 (JP)
(72) Inventor: Ogata, Kazumi, Toyonaka, Osaka 565 (JP); Nagao, Rie, Neyagawa, Osaka 572 (JP); Sakaue, Takahiro, Itami, Hyogo 664 (JP); Ogino, Shinya, 215-1, Senzo 2-chome, Itami, Hyogo 664 (JP); Matsuura, Sachiko, Abeno-ku, Osaka-shi, Osaka 545 (JP)
(74) Representative: Burford, Anthony Frederick

(56) References cited:
- EP-A- 0 027 987
- JP-A-62 145 019
- US-A- 5 122 536
- PATENT ABSTRACTS OF JAPAN vol. 011 no. 371 (C-462) ,3 December 1987 & JP-A-62 145019 (SENJIYU SEIYAKU KK) 29 June 1987,
- PATENT ABSTRACTS OF JAPAN, vol. 011, no. 371 (C-462), 3 December 1987; & JP-A-62 145019

## Description

This invention relates to a useful pharmaceutical composition for the treatment of skin diseases. More particularly, this invention relates to a useful pharmaceutical composition for the treatment of epidermis proliferative diseases, such as psoriasis, ichthyosis, palmoplantar keratoderma and pityriasis rubra pilaris, comprising a phosphate diester compound of Formula I as defined hereinafter or a pharmacologically acceptable salt thereof.

The epidermis or outermost layer of the skin protects the internal environment of the dermis from the external environment. If this epidermis is damaged for whatever cause, it undergoes vigorous growth, thus causing psoriasis which is a proliferative disease of the epidermis. Psoriasis, also known as inflammatory keratosis, is one of the typical diseases of the skin. It forms a singular to numerous lesions varying in size and shape, with intense redness (erythema), slightly raised from the normal skin surface (infiltration), and frequently accompanied by thick scaling. Once psoriasis develops, it is refractory and in most cases persists for the rest of one's life repeating remission and exacerbation.

For the pharmacotherapy of psoriasis steroidal antiinflammatory drugs and immunosuppressive drugs have heretofore been used. Among them, steroidal antiinflammatory drugs are efficacious in psoriasis but induce or aggravate fungal and bacterial infections and cause a variety of adverse reactions such as purpula and peristomatous dermatitis. Therefore, massive or long-term administration of such a steroidal antiinflammatory drug calls for caution with constant attention to the possible onset of such side effects. Immunosuppressant drugs have the disadvantage of inducing renal disorder, hypertension and other side effects. More recently, vitamin D preparations have come into clinical use but are not satisfactory enough in efficacy.

EP-A-0027987 discloses delta-tocopheroles or an ester thereof for the treatment of psoriasis, and US-A-5,122,536 disclose the use of ascorbic acid or an ester thereof for the treatment of psoriasis.

Therefore, in the field of dermatological therapeutics, much research and development work is going on in search of improved drugs for the treatment of epidermis proliferative diseases. The present inventors, who had been investigating the pharmacological profile of certain phosphate diester compounds in detail, discovered that these compounds are of use for the treatment of epidermis proliferative diseases.

According to the present invention, there is provided the use of a phosphoric diester compound of the following Formula 1 or a pharmacologically acceptable salt thereof (hereinafter referred to as the present compounds). 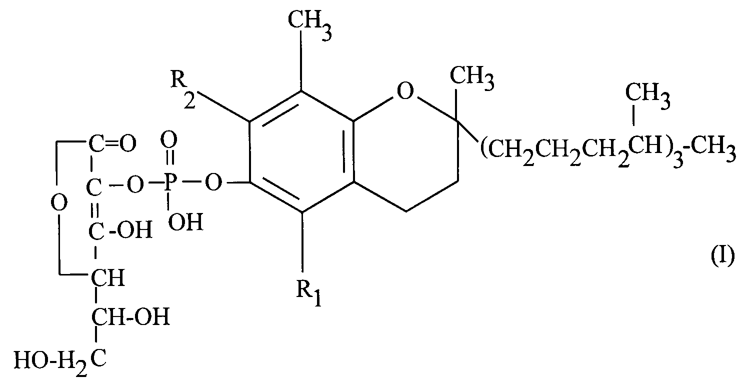 [wherein R₁ and R₂ are the same or different and each represents a hydrogen atom or a methyl group] in the manufacture of a medicament for the treatment of epidermis proliferative diseases.

The present compounds can be synthesized by, inter alia, the processes described in JP-A-2-44478 and JP-A-62 205091.

The present compounds are already known as, for example, anticataract drugs, prophylactic and therapeutic drugs for climacteric disturbance, skin care cosmetic ingredients (JP-A-2-44478), antiinflammatory drugs (JP-A-1-27044), antiulcer drugs (JP-A-63-270626), prophylactic and therapeutic drugs for ischemic organ diseases (JP-A-2-111722), and Maillard reaction inhibitors (JP-A-3-161444).

The present compounds can be either the free compound or a pharmacologically acceptable salt. The pharmacologically acceptable salts include not only alkali metal salts such as sodium salts and potassium salts and alkaline earth metal salts such as calcium salts and magnesium salts but also other salts which are pharmacologically acceptable.

According to the intended use and need, the medicament of this invention may comprise one species or a plurality of species, in a suitable combination, of the present compounds.

The present compounds are very safe with extremely low toxic potential and can, therefore, be used with advantage for the purposes of this invention. For example, the LD₅₀ of L-ascorbyl DL-α-tocopheryl phosphate potassium (abbreviation: EPC-K): is ≥ 5 g/kg p.o. (rats) and ≥ 100 mg/kg i.v. (rats).

The present compounds can be administered orally or otherwise. The dosage form in which the present compounds can be provided includes, for example, ointments, aqueous liquids, gels, tablets, granules, and powders. These dosage forms can be respectively manufactured by established pharmaceutical procedures. In these dosage forms, a variety of additives such as excipients, binders, disintegrators, dispersants, reabsorption promoters, buffers, surfactants, solubilizers, preservatives, emulsifiers, isotonizing agents, stabilizers and pH control agents can be included in appropriate amounts.

In application of the present compounds for the treatment of epidermis proliferative diseases, the dosage depends on the disease, species of compound, the patient's age and body weight, clinical manifestations that must be controlled, and the dosage form. In the case of an ointment or gel for topical application, a preparation containing 0.01 - 5 (w/w) %, preferably 0.05 - 2 (w/w) %, of a present compound can be applied to the affected skin area a few times a day for the average adult. In the case of an aqueous liquid for topical application, a preparation of 0.01 - 5 (w/v) % concentration, preferably 0.05 - 2 (w/v) % concentration, is applied to the affected skin area a few times daily for the average adult. In the case of an oral preparation, 10 mg - 1000 mg per dose can be administered a few times a day for the average adult.

Unless contraindicated, the medicament of this invention may further contain other agents for the treatment of epidermis proliferative diseases and/or other kinds of medicinal substances.

The following Examples and Formulation Examples are intended to describe this invention in further detail.

### Example 1: Assay of efficacy of the present compound in the mouse model of psoriasis vulgaris

The efficacy of EPC-K was assayed in the mouse model of psoriasis vulgaris using 12-o-tetradecanoylphorbol-13-acetate (briefly TPA).

### Test substances:

| | |
|---|---|
| (1) Methanol (control drug) | 20 µl/auricle |
| (2) EPC-K | 500 µg/20 µl/auricle |
| (3) EPC-K | 1000 µg/20 µl/auricle |
| (4) Betamethasone valerate | 25 µg/20 µl/auricle |

### Method:

Male 6-week-old ddY mice purchased from SLC Japan were used.

The entire external surface of the right auricle of each mouse was coated with 20 nmol/40 µl of TPA/methanol, and after 6, 24, 48 and 72 hours, the thickness of the auricle was measured with a dial thickness gauge. The ear swelling rate (%) was then calculated. At 72 hours after application of TPA, the mouse was sacrificed and the right and left auricles were respectively punched out with a 8 mm (dia.) punch. The epidermis of each auricle was peeled off and the epidermal weight was determined. Then, the % gain in epidermal weight of the right auricle relative to the left auricle was calculated.

Each test substance was extemporaneously blended with TPA-methanol and 40 µl of the mixture was applied to the external surface of the right auricle of each mouse. Then, a methanolic solution of the corresponding test substance, 40 µl, was applied once daily for 2 days.

Dunnet's test was used for statistical efficacy evaluation of the test substance.

### Results:

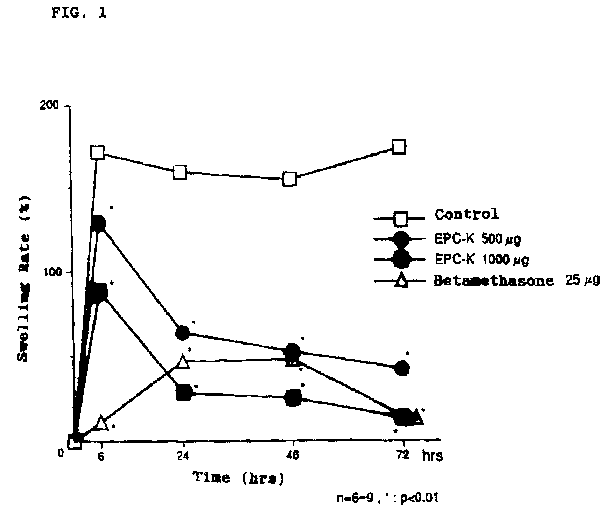

The results are shown in Table 1 and Fig. 1. Table 1 shows inhibitory effect of EPC-K on ear epidermal weight gain in the mouse model of psoriasis vulgaris and Fig 1 is a graph showing the time course of ear swelling rate in the mouse model of psoriasis vulgaris. The abscissa represents time (hours) and the ordinate represents the swelling rate (%).

**Table 1**

| Inhibitory effect of EPC-K on ear epidermal weight gain in the mouse model of psoriasis vulgaris | | | | |
|---|---|---|---|---|
| Group | Control | EPC-K | | Betamethasone Valerate |
| Dose/auricle | | 500 µg | 1000 µg | 25 µg |
| Epidermal weight % gain | 110.9±68.6 | 52.4±40.1* | 21.8±17.4** | 21.0±19.9** |

Each figure in the table represents mean±SD (n=6-9). Significant difference from control: ** p<0.01, * p<0.05.

It is apparent from Table 1 that 500 and 1000 µg of EPC-K inhibited the epidermal weight gain significantly, by 52.8% and 80.3%, respectively. On the other hand, 25 µg betamethasone valerate inhibited the epidermal weight gain significantly by 81.1%, or equipotently as 1000 µg of EPC-K, but it induced body weight loss as a side effect.

In the ear swelling rate, too, EPC-K showed dose-dependent inhibitory effects at all points of time as shown in Fig. 1. On the other hand, 25 µg betamethasone valerate also showed fairly potent efficacy but its effect had been attenuated by 24 and 48 hours after application. Thus, a more potent inhibitory effect was obtained in the group treated with 1000 µg of EPC-K.

It is, therefore, clear that EPC-K is useful for the treatment of psoriasis which is a proliferative disease of the epidermis.

### Formulation Example 1: Ointment

| | |
|---|---|
| EPC-K | 1.0 g |
| Hydrophilic ointment base | to 100 g |

The above components are mixed in a routine manner to provide an ointment.

### Formulation Example 2: Aqeous liquid

| | |
|---|---|
| EPC-K | 0.5 g |
| Glycerin | 1.0 g |
| Propylene glycol | 1.5 g |
| Ethanol | 30 ml |
| Sterilized purified water | to 100 ml |

The above components are mixed in a routine manner to provide an aqueous liquid.

### Formulation Example 3: Gel

| | |
|---|---|
| EPC-K | 0.5 g |
| Carboxyvinyl polymer | 1.0 g |
| Triethanolamine | q.s. |
| Ethanol | 30 ml |
| Sterilized purified water | to 100 ml |
| | pH=7.0 |

The above components are mixed in a routine manner to provide a gel.

### Formulation Example 4: Oral tablet

| | |
|---|---|
| EPC-K | 100 mg |
| Lactose | 75 mg |
| Starch | 20 mg |
| Polyethylene glycol 6000 | 5 mg |

The above components (per tablet) are mixed and compressed in a routine manner to provide tablets. Where necessary, the tablets may be sugar-coated.

## Claims

1. The use of a phosphoric diester compound of the following Formula I or a pharmacologically acceptable salt thereof. 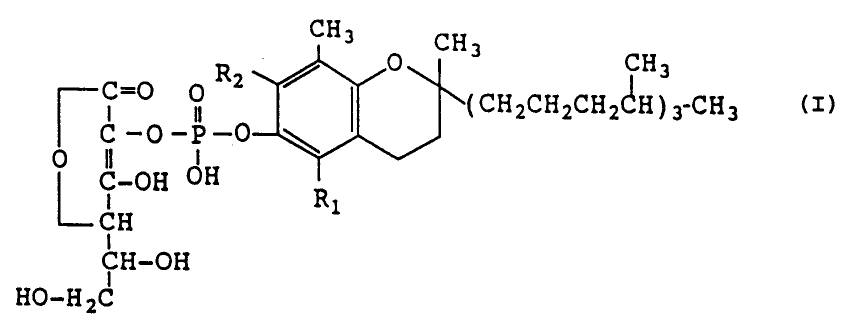 [wherein R₁ and R₂ are the same or different and each represents a hydrogen atom or a methyl group]
in the manufacture of a medicament for the treatment of epidermis proliferative diseases.

2. A use according to Claim 1, wherein the medicament is for topical application.

3. A use according to Claim 2, wherein the medicament is an ointment, an aqueous liquid, or a gel.

4. A use according to Claim 2 or Claim 3, wherein the concentration of said compound or pharmacologically acceptable salt in said medicament is 0.01 - 5 (w/w) %.

5. A use according to Claim 4, wherein the concentration of said compound or pharmacologically acceptable salt in said medicament is 0.05 - 2 (w/w) %.

6. A use according to Claim 1, wherein the medicament is for oral administration.

7. A use according to Claim 5, wherein the amount of said compound or pharmacologically acceptable salt in said medicament is 10 - 1000 mg/unit dose.

8. A use according to any one of the preceding claims, wherein the said compound is L-ascorbyl DL-α-tocopheryl phosphoric acid or a salt thereof.

9. A use according to any one of the preceding claims, wherein the medicament is for treating psoriasis.

10. A use according to any one of claims 1 to 8, wherein the medicament is for treating ichthyosis, palmoplantar keratoderma or pityriasis rubra pilaris.

## Patentansprüche

1. Verwendung einer Phosphorsäurediesterverbindung gemäß der folgenden Formel I oder eines pharmakologisch annehmbaren Salzes davon: 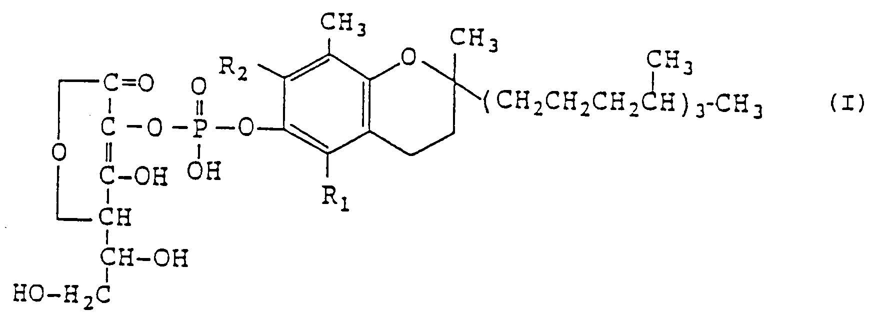 [worin R₁ und R₂ gleich oder verschieden sind und jeweils ein Wasserstoffatom oder eine Methylgruppe bedeuten]
bei der Herstellung eines Arzneimittels zur Behandlung von proliferativen Epidermiserkrankungen.

2. Verwendung nach Anspruch 1, bei der das Arzneimittel durch die topische Verabreichung gekennzeichnet ist.

3. Verwendung nach Anspruch 2, bei der das Arzneimittel eine Salbe, eine wäßrige Flüssigkeit oder ein Gel darstellt.

4. Verwendung nach Anspruch 2 oder Anspruch 3, bei der die Konzentration an der besagten Verbindung oder an dem pharmakologisch annehmbaren Salz in dem Arzneimittel 0,01 bis 5 Gew.-% beträgt.

5. Verwendung nach Anspruch 4, bei der die Konzentration an besagter Verbindung oder an dem pharmakologisch annehmbaren Salz in dem Arzneimittel 0,05 bis 2 Gew.-% beträgt.

6. Verwendung nach Anspruch 1, bei der das Arzneimittel durch die perorale Verabreichung gekennzeichnet ist.

7. Verwendung nach Anspruch 5, bei der die Menge an der besagten Verbindung oder an dem pharmakologisch annehmbaren Salz in dem Arzneimittel 10 bis 1000 mg/Einheitsdosis beträgt.

8. Verwendung nach einem der vorstehenden Ansprüche, bei der die besagte Verbindung die L-Ascorbyl-D,L-α-tocopherylphosphorsäure oder ein Salz davon darstellt.

9. Verwendung nach einem der vorstehenden Ansprüche, bei der das Arzneimittel zur Behandlung von Psoriasis ist.

10. Verwendung nach einem der Ansprüche 1 bis 9, bei der das Arzneimittel zur Behandlung von Ichthyosis, der palmoplantaren Keratodermie oder von Pityriasis rubra pilaris ist.

## Revendications

1. Utilisation d'un composé diester phosphorique de formule suivante I ou d'un de ses sels acceptable d'un point de vue pharmacologique 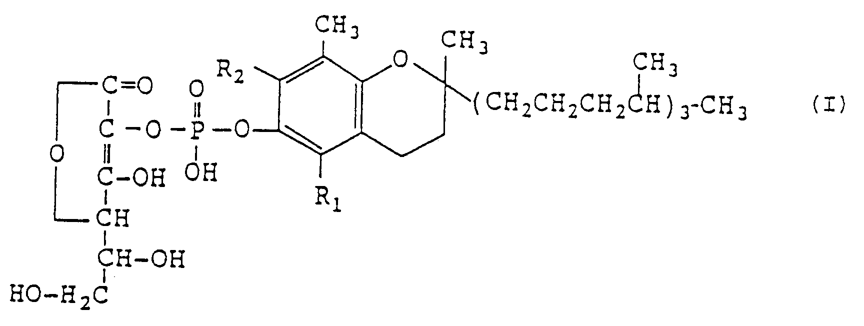 [où R₁ et R₂ sont identiques ou différents et représentent chacun un atome d'hydrogène ou un groupement méthyle]
dans la fabrication d'un médicament pour le traitement de maladies proliférantes de l'épiderme.

2. Utilisation selon la revendication 1, où le médicament est destiné à une application topique.

3. Utilisation selon la revendication 2, où le médicament est un onguent, un liquide aqueux, ou un gel.

4. Utilisation selon l'une des revendications 2 et 3, où la concentration dudit composé ou sel acceptable d'un point de vue pharmacologique dans ledit médicament est de 0,01 à 5 % (p/p).

5. Utilisation selon la revendication 4, où la concentration dudit composé ou sel acceptable d'un point de vue pharmacologique dans ledit médicament est de 0,05 à 2 % (p/p).

6. Utilisation selon la revendication 1, où le médicament est destiné à une administration orale.

7. Utilisation selon la revendication 5, où la quantité dudit composé ou sel acceptable d'un point de vue pharmacologique dans ledit médicament est de 10-1000 mg/dose unitaire.

8. Utilisation selon l'une quelconque des revendications 1 à 7, où ledit composé est l'acide L-ascorbyl-DL-α-tocophérylphosphorique ou un sel de celui-ci.

9. Utilisation selon l'une quelconque des revendications 1 à 8, où le médicament est destiné au traitement du psoriasis.

10. Utilisation selon l'une quelconque des revendications 1 à 9, où le médicament est destiné au traitement de l'ichtyose, de la kératodermie palmo-plantaire et du pityriasis rubra pilaire.
